# EUROPEAN PATENT SPECIFICATION

(11) **EP 2 879 629 B1**
(45) Date of publication and mention of the grant of the patent: **07.09.2016**
(21) Application number: 13748172.7
(22) Date of filing: 29.07.2013
(51) Int. Cl.: A61F 5/451, A61B 10/00

(54) **URINE COLLECTION SYSTEM, APPARATUS AND METHOD**
URINSAMMELSYSTEM, VORRICHTUNG UND VERFAHREN
SYSTEME DE COLLECTE D'URINE, APPAREIL ET PROCEDE

(30) Priority: 31.07.2012 US 201261677840 P
(43) Date of publication of application: 10.06.2015
(73) Proprietor: Covidien LP, Mansfield, MA 02048 (US)
(72) Inventor: MOGHE, Ajit K., Bellingham, MA 02019 (US); ELLIOTT, Chelsey, Northbridge, MA (US); PATEL, Harish A., Norfolk, MA 02056 (US)
(74) Representative: Gray, James
(86) International application number: PCT/US2013/052468
(87) International publication number: WO 2014/022262

(56) References cited:
- WO-A1-96/08219
- WO-A2-2006/044621
- DE-A1- 19 915 454
- US-A- 5 919 146
- US-A1- 2007 203 463

## Description

### BACKGROUND

### 1. Technical Field

The present disclosure relates generally to fluid collection systems, and more particularly, to vented fluid collection systems having a clamping tube.

US 5,919,146 A is regarded as the closest prior art.

### 2. Background of Related Art

Urine collection systems for collecting urine from a catheterized patient are well known in the art. Such systems typically include a drain tube having a first end connected to a urinary catheter of a catheterized patient and a second end connected to a urine collection bag. The urine collection bag includes an outlet port for draining fluid from the bag. Typically an in-line adapter is provided for connecting the urinary catheter to the drain tube. The adapter may include a sampling port for providing access to the urine flow upstream of the urine collection bag to facilitate testing of the urine.

In known systems, a vent may be provided in line with the drain tube and the adapter. Typically, the in-line vent is connected to the downstream side of the sampling port adapter and functions to normalize the pressure in the collection system and prevent lesions within a patient's bladder due to suction created by drainage of the urine collection bag. Often, the in-line vent is close-coupled to the sampling port, for example, the upstream side of the in-line vent may be immediately adjacent the downstream side of the sampling port.

Generally, the fluid path in which the sampling port is located must be temporarily sealed downstream of the sampling port to allow the sampling port to be used. Hemostats or other types of clamps are employed to temporarily seal the urine collection drain line. However, where an in-line vent is employed immediately adjacent the sampling port, the drain line downstream of the sampling port cannot be temporarily sealed because the first available location for clamping is downstream of the vent. Because the vent is open to atmospheric pressure, the vent will become saturated with urine when a clamp is applied downstream.

### SUMMARY

According to various aspects, a fluid collection system includes a compressible tube located between a sampling port adapter and an in-line vent. In further aspects, the fluid collection system includes a fluid flow path configured to accelerate fluid flow downstream of a location where a clamp is secured to temporarily stop fluid flow. In some embodiments, a fluid channel included in the compressible tube is configured to accelerate fluid flow following a release of a clamp applied to the compress the tube and temporarily seal the fluid channel of the compressible tube.

In accordance with one aspect, a urine collection system includes a collection bag, an in-line vent positioned upstream of the collection bag and being fluidly coupled to the collection bag, a sampling port adapter positioned upstream of the in-line vent, and a compressible tube defining a fluid channel fluidly connecting the sampling port adapter and the in-line vent. In some embodiments, the collection bag defines a fluid reservoir including a fluid inlet and a fluid outlet, the in-line vent includes an outlet end, the sampling port adapter is adapted to be fluidly coupled to a urinary catheter and the compressible tube is clampable to seal the fluid channel.

According to one embodiment, an inside diameter of the fluid channel is larger at a downstream end of the fluid channel than the inside diameter at an upstream end of the fluid channel. According to a further embodiment, the compressible tube includes a compressible portion which defines a tapered fluid channel providing a fluid flow path with an increasing diameter in a downstream direction.

According to another aspect, an apparatus is provided for sealing a fluid channel in a urine collection system including each of a sample port fitting configured to allow access to the fluid channel and a vent configured to couple the fluid channel to atmospheric pressure. In some embodiments, the apparatus includes a compressible tube having a first end configured to couple to an outlet end of the sample port fitting, the first end having a first inside diameter, and a second end configured to couple to an inlet end of the vent, the second end having a second inside diameter, the second inside diameter greater than the first inside diameter. In accordance with these embodiments, the compressible tube defines the fluid channel between the outlet end of the sample port fitting and the upstream end of the vent and the compressible tube is configured to compress to stop the flow of fluid in the fluid channel in response to clamping pressure applied at a portion of the compressible tube located between the first end and second end.

According to one embodiment, the first end includes an outside diameter sized to be received within the sample port fitting to provide an air-tight connection with the first end coupled to the sample port fitting, and the second inside diameter is sized to fit around an outside surface of the upstream end of the vent to provide an air-tight connection with the second end coupled to the vent.

According to yet another aspect, a method of temporarily sealing a fluid channel in a urine collection system is provided where the urine collection system includes a sample port fitting and an in-line vent located downstream of the sample port fitting in the fluid channel. In accordance with some embodiments: a compressible tube is located in the fluid channel between the sample port fitting and the in-line vent, where the compressible tube includes outside walls, an upstream fluid channel cross-sectional area located at an upstream end of the compressible tube and a downstream fluid channel cross-sectional area located at a downstream end of the compressible tube; sufficient pressure directed radially inward on the outside walls is applied to compress the compressible tube and create a temporary seal to stop a flow of urine in the fluid channel; and the pressure is released to remove the seal and allow urine temporarily blocked by the seal to drain from the fluid channel. In accordance with these embodiments, the downstream fluid channel cross-sectional area is greater than the upstream fluid channel cross-sectional area.

According to further embodiments, the compressible tube is selected to provide a ratio of the downstream fluid channel cross-sectional area to the upstream fluid channel cross-sectional area that accelerates a rate of drainage of urine from the compressible tube when the pressure is released relative to the rate of drainage of urine provided when a compressible tube having a constant fluid channel cross-sectional area is located in the fluid channel between the sample port fitting and the in-line vent. In accordance with one embodiment, the ratio is selected to accelerate the rate of drainage by at least a factor of two.

According to still another aspect, a urine collection system includes a collection bag defining a fluid reservoir including a fluid inlet and a fluid outlet, a drain line fluidly coupled to the fluid inlet, an in-line vent positioned upstream of the collection bag and being fluidly coupled to the collection bag by the drain line, a sampling port adapter positioned upstream of the in-line vent and being adapted to be fluidly coupled to a urinary catheter, the sampling port adapter including an upstream end having an upstream flow-path diameter and a downstream end having a downstream flow-path diameter, and a compressible tube defining a fluid channel fluidly connecting the sampling port adapter and the in-line vent, the compressible tube being clampable to seal the fluid channel, the compressible tube including an upstream end having an upstream flow-path diameter and a downstream end having a downstream flow-path diameter. According to some embodiments, the upstream flow-path diameter of the compressible tube is at least as great as the downstream flow-path diameter of the sampling port adapter, and the downstream flow-path diameter of the compressible tube is greater than the upstream flow-path diameter of the compressible tube.

In accordance with one embodiment, the in-line vent line includes an upstream end having an upstream flow-path diameter and a downstream end having a downstream flow-path diameter, the downstream end of the compressible tube is configured to couple to the upstream end of the in-line vent, the upstream flow-path diameter of the in-line vent is at least as great as the downstream flow-path diameter of the compressible tube, and a minimum diameter of a flow-path provided by the in-line vent is no less than the upstream flow-path diameter of the in-line vent.

In accordance with a further embodiment, the drain line includes an upstream end having an upstream flow-path diameter and a downstream end having a downstream flow-path diameter. In accordance with this embodiment, the downstream end of the in-line vent is configured to couple to the upstream end of the drain line, the upstream flow-path diameter of the drain line is at least as great as the downstream flow-path diameter of the in-line vent, and a minimum diameter of a flow-path provided by the drain line is no less than the upstream flow-path diameter of the drain line.

In accordance with still another aspect, a method of urine collection is provided with a urine collection system that defines a flow path and comprises components including a sample port fitting defining a first region of a fluid channel for urine flow, an in-line vent located downstream of the sample port fitting and defining a second region of the fluid channel, a clamping tube defining a region of the fluid channel between the sample port fitting and the in-line vent, and a drain line defining a region of the fluid channel between the in-line vent and a collection bag. According to some embodiments, the method includes: providing a minimum diameter of the fluid channel defined by each of the sample port fitting, the in-line vent, the clamping tube and the drain line, respectively, at an inlet to each of the respective components; and providing a diameter of the fluid channel defined at the inlet of each of the respective components to be at least as large as a maximum diameter of the fluid channel defined by the respective component located immediately upstream in the flow path.

As used herein, the terms "tube" refers to a hollow body that includes a longitudinal fluid channel for holding and conveying fluid. It should be understood that the fluid channel in a tube can be formed in any one of a variety of geometric shapes when viewed in cross-section. Accordingly, the shape of the cross-section when viewed in profile can be but may not be circular depending on the embodiment.

### BRIEF DESCRIPTION OF THE DRAWINGS

Various embodiments of the presently disclosed fluid collection system are described herein with reference to the drawings, wherein:
FIG. 1 is a perspective view of the presently disclosed fluid collection system in accordance with one or more aspects;
FIG. 2 is a side perspective view, with parts separated, of a sampling port adapter, a compressible tube, and an in-line vent of the fluid collection system illustrated in FIG. 1;
FIG. 3 is a side perspective view of the sampling port adapter, compressible tube, and in-line vent assembled and connected between the drain tube and the catheter in accordance with one or more aspects; and
FIGS. 4A-4D illustrate alternate embodiments of the compressible tube of the fluid collection system shown in FIG. 1.

### DETAILED DESCRIPTION

Embodiments of the presently disclosed fluid collection system will now be described in detail with reference to the drawings wherein like reference numerals designate identical or corresponding elements in each of the several views.

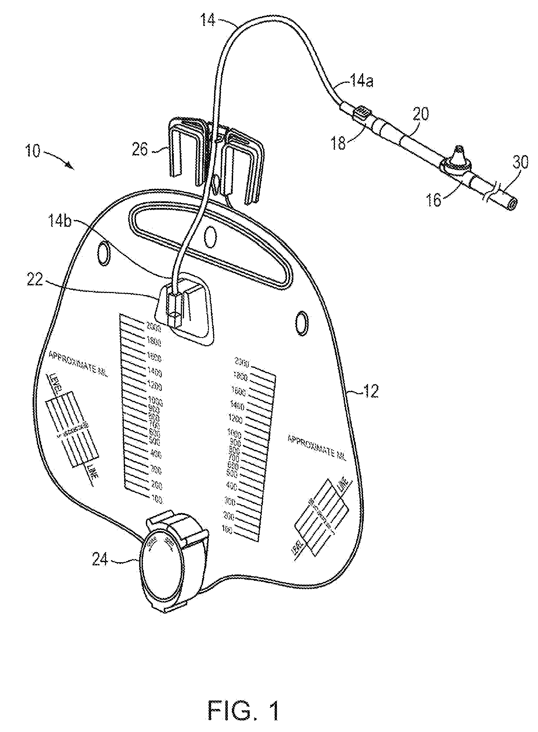

FIGS. 1-3 illustrate one embodiment of the presently disclosed urine collection system which is shown generally as 10. Urine collection system 10 includes a urine collection bag 12, a drain tube 14, a sampling port adapter 16, an in-line vent 18, and a compressible tube 20. Urine collection bag 12 may assume a variety of configurations depending on the embodiment. In one embodiment, urine collection bag 12 includes an anti-reflux valve 22 and a discharge valve 24. A support member, such as support member 26, may be provided to releasably secure urine collection bag 12 to a support structure (not shown), such as a bedframe. One such urine collection bag is disclosed in U.S. Patent Application Publication No. 2007/0203463 ("the '463 publication") . Although not shown, urine collection system may also include a urine meter such as described in U.S. Patent No. 7,645,968 .

Referring again to the embodiment illustrated in FIG. 1, the urine collection system 10 is used to collect fluid, e.g., urine, from a catheterized patient. Fluid flows from a bladder of the patient for collection in the collection bag 12, first via a urinary catheter 30, then through the sampling port adapter 16, through the compressible tube 20, followed by the in-line vent 18, and then into the drain tube 14. The drain tube 14 includes an inlet end 14a and an outlet end 14b. Fluid flows from the drain tube 14, through the anti-reflux valve 22 and into the urine collection bag 12. Fluid can be drained from collection bag 12 via the discharge valve 24.

According to various embodiments, the components of the urine collection system, for example, the sample port adapter 16, the compressible tube 20, the in-line vent 18 and the drain line 14 provide an unobstructed fluid channel from the catheter 30 to the collection bag 12. In these embodiments, each of the components is constructed to provide a fluid channel having a cross-sectional area for fluid flow, respectively. In some embodiments, the cross-sectional area for fluid flow can increase from an inlet end to an outlet end of any one of the individual components, for a plurality of the components or for each of the components located in the fluid flow path between the catheter 30 and the collection bag 12.

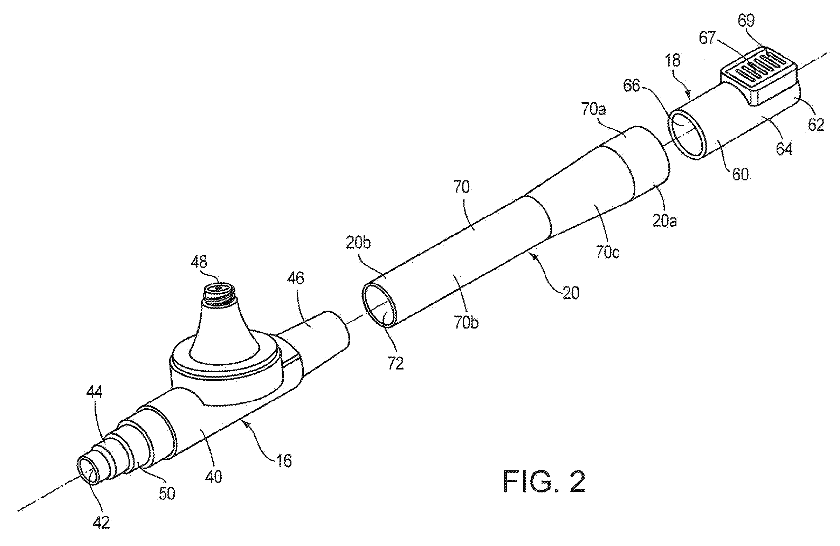

Referring to FIGS. 2-3, the sampling port adapter 16 may include any of a variety of different access ports which facilitate withdrawal of a sample of fluid from the system 10 upstream of the collection bag 12 for testing. Examples of known sampling ports are described in the '463 publication. Further, although illustrated as a fitting including both a sample port and an adapter for connecting the fluid flow path to the catheter, other embodiments may provide a separate adapter to connect to the catheter and a fitting including the sample port located downstream of the adapter in the fluid flow path.

Referring to the embodiment illustrated in FIGS. 1 and 2, the sampling port adapter 16 includes a body portion 40 which defines a longitudinal channel 42. The body portion 40 includes an inlet end 44, an outlet end 46 and an access port 48 configured to allow withdrawal of a fluid sample from the longitudinal channel 42. In the illustrated embodiment, the inlet end 44 of the body portion 40 is tapered and is dimensioned to be received within a downstream end of the urinary catheter 30. According to various embodiments, the body portion 40 includes a rigid or substantially rigid body manufactured, for example, from a molded plastic such as ABS or PVC.

In the embodiment illustrated in FIG. 2, the compressible tube 20 includes an inlet end 20a, an outlet end 20b and a body 70. The outlet end 46 of body portion 40 is tapered and is dimensioned to be received within the inlet end 20b of the compressible tube 20. In some embodiments, the inlet end 44 and the outlet end 46 of the body portion 40 can be secured by an adhesive to either or both of the catheter 30 and the compressible tube 20, respectively, to provide an airtight, non-removable attachment. Depending on the embodiment, the outlet end 46 and/or the inlet end 44 of the body portion 40 may include ribs, protrusions, bumps or the like to more securely fasten the sampling port adapter 16 between the urinary catheter 30 and the compressible tube 20. In the illustrated embodiment, the inlet end 44 includes annular steps 50 to assist in securing the sampling port adapter 16 between urinary catheter 30 and the compressible tube 20. Alternately, other fastening techniques (for example, annular compression clamps) may be used alone or in combination with the preceding to secure the sampling port adapter 16 to each of the drain tube 14 and the compressible tube 20.

In the illustrated embodiment, the in-line vent 18 is positioned between the outlet end 20a of the compressible tube 20 and the inlet end 14a of drain tube 14. According to this embodiment, the in-line vent 18 includes an inlet end 60, an outlet end 62, and a housing 64 defining a longitudinal channel 66 which permits fluid flow through the housing 64 between the inlet end 60 and the outlet end 62. Further, the vent housing 64 supports a venting structure 67. According to various embodiments, the in-line vent 18 includes a substantially rigid body manufactured, for example, from a molded plastic such as ABS or PVC.

The in-line vent 18 is dimensioned to engage each of the compressible tube 20 and the drain tube 14. In the illustrated embodiment, the relative dimensions of the outlet end 20a of the compressible tube 20 and the in-line vent 18 allow the in-line vent 18 to be received within the outlet end 20a of the tube 20. Further, in the illustrated embodiment, the relative dimensions of the inlet end 14a of the drain tube 14 and the in-line vent 18 allow the inlet end 14a of the drain tube 14 to be received within the in-line vent 18. In some embodiments, the flexibility of the material of construction of the compressible tube 20 and the drain tube 14, respectively, facilitate the attachment of the respective tubes to the in-line vent 18 because the tube material will yield to pressure and stretch to fit around a connection or squeeze to fit within a connection. In accordance with these embodiments, a fluid-tight connection to the in-line vent 18 is achieved.

As should be apparent to those of ordinary skill in the art in view of the disclosure provided herein, in other embodiments, the relative dimensions of the compressible tube 20 and the in-line vent 18 can be configured to allow the compressible tube 20 to be received within the in-line vent 18. Similarly, the relative dimensions of the drain tube 14 and the in-line vent 18 can be configured to allow the in-line vent 18 to be received within the inlet end 14a of the drain tube 14.

According to some embodiments, an adhesive can be used to ensure that the in-line vent 18 is securely attached to the compressible tube 20 and the drain tube 14, respectively, in an airtight and non-removable manner. Alternately, other fastening techniques may be used alone or in combination with the preceding to secure the in-line vent 18 to the compressible tube 20 and the drain tube 14, for example, annular pressure clamps can be used.

In some embodiments, the venting structure 67 permits air to enter the collection system 10 to prevent siphoning of fluid from a patient's bladder during emptying of the collection bag 12. The in-line vent 18 also functions to minimize back pressure in collection system 10 especially when the drain tube 14 hangs below collection bag 12. In addition, the in-line vent 18 helps to normalize the pressure in the collection system 10 and helps prevent lesions in a patient's bladder caused by suction events in the drainage tubing.

The in-line vent 18 may assume a variety of known configurations. In one embodiment disclosed in the '463 publication, the in-line vent 18 includes an oleophobic expanded PTFE membrane 69 supported within housing 64 which is formed from a substantially rigid plastic such as PVC or ABS. The oleophobic membrane sold under the trademark GORE-TEX®, available from W.L. Gore & Associates, Inc. of Newark, Delaware, USA is one such oleophobic material. Alternately, other materials of construction can be used.

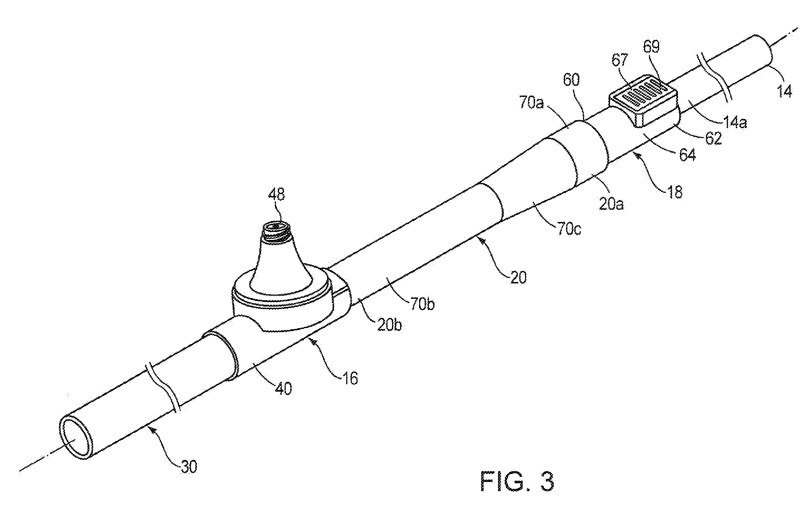

Referring to the embodiment illustrated in FIGS. 2 and 3, in one embodiment the compressible tube 20 includes a body 70 which defines a fluid channel 72. In accordance with the illustrated embodiment, the body 70 includes an upstream portion 70b which is substantially cylindrical, a downstream portion 70a which is substantially cylindrical and a tapered portion 70c which increases in diameter from the upstream portion 70b towards the downstream portion 70a near the vent 18. According to some embodiments, the compressible tube 20 includes the tapered design to provide a tapered fluid channel to increase the diameter of the fluid channel 72 between the inlet end 20b and the outlet end 20a of the tube 20 (between the sampling port adapter 16 and the in-line vent 18). According to an alternate embodiment, the compressible tube 20 has a constant diameter fluid channel and does not include a taper. In various embodiments, the body 70 is formed from a resilient, compressible material, such as any of extruded PVC material, polyurethane, natural rubber or synthetic rubber which can be clamped to seal the fluid channel 72 as discussed in more detail below.

In embodiments that include a tapered design, the increasing diameter of the fluid channel 72 helps relieve the resistance to fluid flow as per Bernoulli's principle. The preceding allows the urine to drain from the compressible tube 20 more quickly because the larger diameter results in a decrease in fluid pressure in the fluid channel 72 of the compressible tube 20. As will be apparent to those of ordinary skill in the art in view of the disclosure provided herein, the increased diameter results in an increased cross-sectional area of the fluid channel 72.

In one embodiment, the compressible tube 20 provides an increase in a diameter of the fluid channel 72 of at least 1mm between the inlet end 20b and the outlet end 20a. According to a further embodiment, the compressible tube 20 provides an increase in a diameter of the fluid channel 72 of at least 1cm between the inlet end 20b and the outlet end 20a. Alternately, other increases in fluid-channel size and rates of increase of the inside diameter of fluid channel 72 are envisioned. In use, an increase in diameter of the compressible tube 20 between the inlet end 20b and the outlet end 20a assists in a relieving the fluid flow backup that would otherwise occur following a release of clamping pressure applied to temporarily seal the urine collection system.

According to one embodiment, the relative dimensions of the in-line vent 18 and the outlet end 20a of the compressible tube 20 allow a connection of the vent 18 and the tube 20 without any reduction in a cross-sectional area of the flow channel provided at a downstream end of the tapered region 70c of the tube 20.

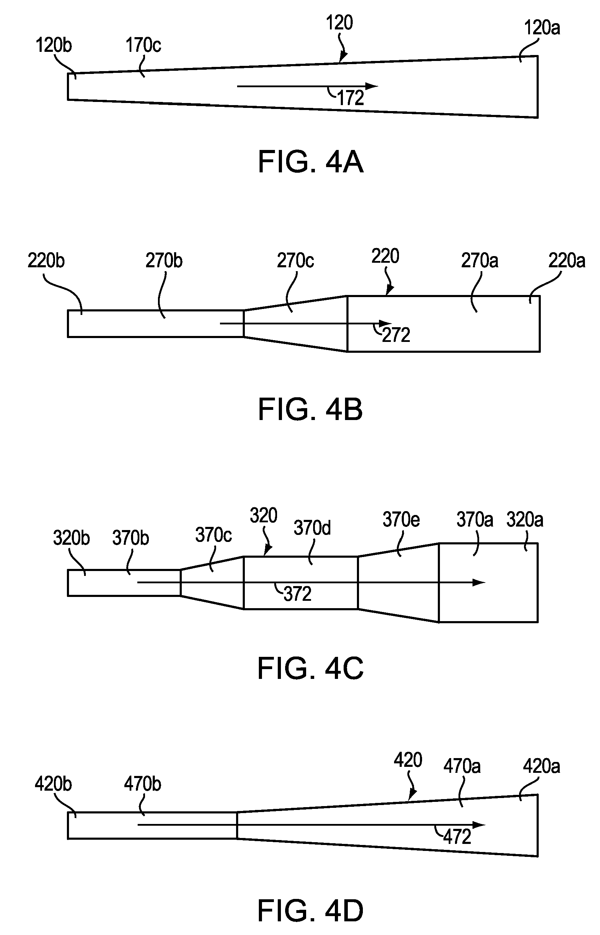

Although the compressible tube 20 in FIGS. 1-3 illustrates cylindrical upstream and downstream portions 70b and 70a, respectively, which are located at opposite ends of the tapered portion 70c, other configurations that provide an increase in cross-sectional area of the fluid channel from the inlet end 20b to the outlet end 20a are possible in other embodiments. Referring to FIG. 4A, according to another embodiment, a compressible tube 120 includes an outlet end 120a, an inlet end 120b and a tapered portion 170c. The compressible tube 120 includes a hollow central region that forms a longitudinal fluid channel for fluid flow in a direction 172 within the compressible tube 120 from the inlet end 120b to the outlet end 120a. In one embodiment, the dimensions of the longitudinal fluid channel are tapered such that a diameter of the fluid channel increases in proportion to the increase in outside diameter of the tapered portion 170c in a direction of fluid flow. In the embodiment illustrated in FIG. 4A, the tapered region 170c uniformly increases in diameter from the inlet end 120b to the outlet end 120a of the compressible tube 120. The preceding results in the longitudinal fluid channel having a cross-sectional area which uniformly increases in diameter from the inlet end 120b to the outlet end 120a of the compressible tube 120.

Referring to FIG. 4B, according to another embodiment, a compressible tube 220 includes an outlet end 220a, an inlet end 220b, a downstream portion 270a, an upstream portion 270b and a tapered portion 270c. The compressible tube 220 also includes a hollow central region that forms a longitudinal fluid channel for fluid flow in a direction 272 within the compressible tube from the inlet end 220b to the outlet end 220a. According to this embodiment, the tapered portion 270c provides the longitudinal fluid channel with a cross-sectional area which increases in diameter from the upstream portion 270b to the downstream portion 270a of the compressible tube 220. However, according to this embodiment, the tapered portion 270c is centrally located along the longitudinal axis of the compressible tube 220 while each of the upstream portion 270b and the downstream portion 270a have a constant diameter, respectively. According to the illustrated embodiment, the upstream portion 270b has a constant diameter equal to a diameter at the upstream end of the tapered region 270c while the downstream region 270a has a constant diameter equal to a diameter at the downstream end of the tapered region 270c.

Referring to FIG. 4C, in accordance with other embodiments, a compressible tube can include a plurality of tapered portions. In the illustrated embodiment, a compressible tube 320 includes an outlet end 320a, an inlet end 320b, a downstream portion 370a, an upstream portion 370b, a first tapered portion 370c, a central portion 370d and a second tapered portion 370e. The compressible tube 320 also includes a hollow central region that forms a longitudinal fluid channel for fluid flow in a direction 372 within the compressible tube from the inlet end 320b to the outlet end 320a. According to this embodiment, the tapered portion 370c provides the longitudinal fluid channel with a cross-sectional area which increases in diameter from a diameter of the upstream portion 370b to a diameter of the central portion 370d. Further, the tapered portion 370e provides the longitudinal fluid channel with a cross-sectional area which increases in diameter from the diameter of the central portion 370d to a diameter of the downstream portion 370a.

Thus, moving from the inlet end 320b to the outlet end 320a in a direction of the flow channel 372, the relative cross-sectional area of the longitudinal fluid channel of the compressible tube 320 increases: a) from the upstream portion 370b which has the smallest diameter, to the first tapered portion 370c; b) along the first tapered portion 370c which increases in diameter from the diameter of the upstream portion 370b to a diameter of the central region 370d; and then c) to the second tapered portion 370e which increases in diameter from the diameter of the central region 370d to a diameter of the downstream portion 370a. As will be apparent to those of ordinary skill in the art in view of the disclosure provided herein, the overall increase in diameter (and corresponding increase in cross-sectional area) of the longitudinal fluid channel of the compressible tube can be achieved with any quantity of tapered portions, tapered portions having varying lengths, and with tapered portions having varying rates of increasing diameter, provided they can fit within the length of the compressible tube and provide upstream diameters and downstream diameters that correspond to those required for connection to the sampling port adapter 16 and the in-line vent 18, respectively.

Referring to FIG. 4D, according to a further embodiment, a compressible tube 420 includes an outlet end 420a, an inlet end 420b, a tapered portion 470a and a constant diameter portion 470b. The compressible tube 420 also includes a hollow central region that forms a longitudinal fluid channel for fluid flow in a direction 472 within the compressible tube from the inlet end 420b to the outlet end 420a. According to the illustrated embodiment, the tapered portion 470c provides the longitudinal fluid channel 472 with an internal diameter (and corresponding cross-sectional area) which increases from the smaller diameter of the inlet end 420b to the larger diameter of the outlet end 420a of the compressible tube 420.

According to another embodiment, a combination of a constant diameter portion and a tapered portion can be employed with the tapered portion located upstream of the constant diameter portion. According to this embodiment, the tapered portion has an inlet end configured to attach to an outlet of a sample port adapter while the constant diameter portion has a diameter (and corresponding cross-sectional area) that equals the diameter found at the downstream end of the tapered portion.

In further embodiments, the increase in diameter (and corresponding increase in cross-sectional area) of the compressible tubes 120, 220, 320, and 420 is provided without a taper. Instead the compressible tube can include one or more step-increases in the inside diameter of the compressible tube. Referring to FIG. 4D as one example, the change in diameter from the diameter provided at the inlet end 420b (the diameter of the constant diameter portion 470b) to the diameter provided at the outlet end 420a can instead occur at a single location along the longitudinal axis of the compressible tube. In further embodiments, the compressible tube can include a plurality of step-increases to achieve a desired overall increase in diameter of the flow channel from the upstream end of the compressible tube to the downstream end of the compressible tube.

According to some embodiments, the cross-sectional area of the fluid channel in the compressible tube has a minimum value at the inlet of the compressible tube. In further embodiments of the preceding, the cross-sectional area of the fluid channel at the outlet of the compressible tube equals a maximum cross-sectional area of the fluid channel of the compressible tube. Further, in some embodiments in which other components include fluid channels having increasing cross-sectional areas at an outlet relative to an inlet of the component, the cross-sectional area of the fluid channel in the component can have a minimum value at the inlet of the component and a maximum value at the outlet of the component.

Each of the compressible tubes 120-420 illustrated in FIGS. 4A-4D includes a compressible body which defines a fluid channel which increases in diameter from its upstream end to its downstream end. The body is formed of a material which can be compressed such that the tube segment defines a clamping area which is accessible to seal flow through fluid channel of the body using any of a variety of known medical clamping devices. Although multiple embodiments are illustrated, many other configurations are envisioned.

In one embodiment, the compressible tube 20 is transparent to facilitate visualization of fluid therein. The compressible tube 20 may be tinted, e.g., a blue tint, to provide an easily identifiable clamping surface of the fluid collection system. In addition, the internal surface of the compressible tube 20 (the walls of the longitudinal fluid channel) may be provided with a non-leachable lubricious coating or characteristic to minimize surface tension within the tube and improve the flow characteristics of the system 10.

In use, a clinician may wish to interrupt flow above the in-line vent 18 to provide access to the sampling port adapter 16 by clamping the compressible tube 20 using any of a variety of known surgical clamps (not shown) to isolate the vent 18, drain tube 14 and collection bag 12 from the sampling port adapter 16 and the urinary catheter 30 (FIG. 1).

After the sampling port adapter 16 has been accessed, the clamp can be removed from the compressible tube 20 to allow fluid flow from the patient's bladder to the collection bag 12 to resume. By providing an increase in diameter of the compressible tube 20 between the inlet end 20b of the tube 20 and the outlet end 20a of the tube 20, surface tension on the fluid is more easily overcome. Thus, fluid blocked from flowing in a clamping operation drains more quickly when the clamp is released such that normal fluid flow within system 10 can immediately resume.

In an alternative embodiment not shown, the in-line vent 18 can be mounted adjacent to the collection bag 12 on the outlet end 14b of the drain tube 14 and the compressible tube is not used. According to this embodiment, the drain tube is configured to be clamped to seal fluid flow through the drain tube. Further, in this and other embodiments, the inside diameter of the drain tube 14 can increase between the inlet end 14a of drain tube 14 and the outlet end 14b to improve the flow characteristics of the system as discussed in more detail below.

Although FIGS. 1-4D illustrate embodiments in which an increasing downstream diameter is provided in the fluid flow channel located in the region between the sampling port adapter 16 and the in-line vent 18, a similar approach can be applied to other regions of the urine collection system. According to these embodiments, urine drainage occurs more efficiently because the increasing downstream diameter of the fluid channel (and corresponding increasing cross-sectional area) accelerate the fluid flow the further downstream the urine is in the flow path from the catheter 30 to the collection bag 12.

According to one embodiment, in addition to the compressible tube 20, the drain line 14 includes an increased diameter flow channel at the outlet end 14b relative to the inlet end 14a. In one embodiment, the increase occurs with a continuously increasing diameter tapered flow channel. In other embodiments, the increased diameter flow channel is a result of one or more discrete tapered regions located in the drain line 14. In another embodiment, the increased diameter flow channel is a result of one or more step-changes in diameter in the drain line 14. According to other embodiments, rigid or semi-rigid components such as the sampling port adapter 16 and the in-line vent 18 are also manufactured to include an increased diameter flow channel at the downstream end of the component relative to the upstream end of the component. According to one embodiment, the fluid flow channel provided from the inlet 44 of the sampling port adapter to the outlet end 14b of the drain line includes a continuously increasing diameter.

Although described herein with reference to fluid flow channels having a circular cross section, the approaches described herein can be employed with fluid flow channels having other shapes. As just one example, the approaches described herein can be applied with fluid flow channels having parallel walls.

Persons skilled in the art will understand that the devices and methods specifically described herein and illustrated in the accompanying drawings are nonlimiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A urine collection system (10) comprising:
a collection bag (12) defining a fluid reservoir including a fluid inlet and a fluid outlet;
an in-line vent (18) positioned upstream of the collection bag and being fluidly coupled to the collection bag, the in-line vent including an outlet end (62);
a sampling port fitting (16) positioned upstream of the in-line vent and being adapted to be fluidly coupled to a urinary catheter (30); and
a compressible tube (20, 120, 220, 320, 420) defining a fluid channel (72, 172. 272, 372, 472) fluidly connecting the sampling port fitting and the in-line vent, the compressible tube being clampable to seal the fluid channel, **characterized in that**
an inside diameter of the fluid channel defined by the compressible tube is larger at a downstream end (70a, 120a, 220a, 320a, 420a) of the fluid channel than the inside diameter at an upstream end (70b, 120b, 220b, 320b, 420b) of the fluid channel.

2. The urine collection system (10) of claim 1, wherein the outlet end (62) of the in-line vent (18) is connected to a drain line (14) and the drain line is connected to the collection bag (12).

3. The urine collection system (10) of claim 1, wherein the compressible tube (20, 120, 220, 320, 420) includes a compressible portion which defines a tapered fluid channel providing a fluid flow path with an increasing diameter in a downstream direction.

4. The urine collection system (10) of claim 3, wherein an inside surface of the compressible tube (20, 120, 220, 320, 420) including the compressible portion which defines the tapered fluid channel is treated with a non-leachable lubricious coating.

5. The urine collection system (10) of claim 3, wherein the compressible tube (20, 120, 220, 320, 420) including the compressible portion which defines the tapered fluid channel is colored to provide an easily identifiable clamping surface.

6. The urine collection system (10) of claim 5, wherein the fluid channel (72, 172, 272, 372, 472) of the compressible tube (20, 120, 220, 320, 420) includes an upstream cylindrical portion and a downstream cylindrical portion, and
wherein the compressible portion which defines the tapered fluid channel is located between the upstream cylindrical portion and the downstream cylindrical portion.

7. The urine collection system (10) of claim 1, wherein the diameter of the fluid channel (72, 172, 272, 372, 472) of the compressible tube (20, 120, 220, 320, 420) increases uniformly from the upstream end to the downstream end.

8. The urine collection system (10) according to claim 1, wherein the compressible tube (320) includes a plurality of compressible portions (370c, 370e) which define tapered fluid channels, respectively, each of the tapered fluid channels providing a fluid flow channel with an increasing diameter in a downstream direction.

9. The urine collection system (10) according to claim 8, wherein the compressible tube (320) includes a respective cylindrical portion (370d) which defines a cylindrically-shaped fluid flow channel to separate each of the plurality of compressible portions.

10. An apparatus for sealing a fluid channel in a urine collection system (10) including each of a sample port fitting (16) configured to allow access to the fluid channel and a vent (18) configured to couple the fluid channel to atmospheric pressure, the apparatus comprising:
a compressible tube (20, 120, 220, 320, 420) having a first end (70a, 120a, 220a, 320a, 420a) configured to couple to an outlet end (46) of the sample port fitting, the first end having a first inside diameter, and a second end (70b, 120b, 220b, 320b, 420b) configured to couple to an inlet end (60) of the vent, the second end having a second inside diameter, the compressible tube defining the fluid channel between the outlet end of the sample port fitting and the upstream end of the vent,
wherein the compressible tube is configured to compress to stop the flow of fluid in the fluid channel in response to clamping pressure applied at a portion of the compressible tube located between the first end and second end, **characterized in that**
the second inside diameter is greater than the first inside diameter.

11. The apparatus of claim 10, wherein the first end (70a, 120a, 220, 320a, 420a) includes an outside diameter sized to be received within the sample port fitting (16) to provide an air-tight connection with the first end coupled to the sample port fitting, and
wherein the second inside diameter is sized to fit around an outside surface of the upstream end of the vent (18) to provide an air-tight connection with the second end coupled to the vent.

12. The apparatus of claim 10, wherein the compressible tube (20, 120, 220, 320, 420) includes at least one tapered portion (70c, 170c, 270c, 370c, 370e, 470a).

13. The apparatus of claim 12, wherein the compressible tube (120) includes a continuous taper from the first end to the second end.

14. The apparatus of claim 12, wherein the compressible tube (320) includes a plurality of tapered portions (370c, 370e).

15. The apparatus of claim 14, wherein the compressible tube (320) includes a constant diameter region (370d) located between and immediately adjacent two of the plurality of tapered portions.

16. The apparatus of claim 12, wherein the compressible tube (20, 120, 220, 320, 420) is sized and configured to couple to the vent (18) in an air tight manner while at least maintaining a maximum inside diameter of the compressible tube.

17. The apparatus of claim 10, wherein the second inside diameter is greater than the first inside diameter by at least 1 mm.

18. The apparatus of claim 10, wherein the first inside diameter is a minimum diameter of the fluid channel defined by the compressible tube (20, 120, 330, 320, 420).

19. The apparatus of claim 18, wherein the first inside diameter is configured to be at least equal to a diameter of the fluid channel defined by the sample port fitting (16).

20. A method of temporarily sealing a fluid channel in a urine collection system (10) including a sample port fitting (16) and an in-line vent (18) located downstream of the sample port fitting in the fluid channel, the method comprising:
locating a compressible tube (20, 120, 220, 320, 420) in the fluid channel between the sample port fitting (16) and the in-line vent (18), the compressible tube including outside walls, an upstream fluid channel cross-sectional area located at an upstream end (70b, 120b, 220b, 320b, 420b) of the compressible tube and a downstream fluid channel cross-sectional area located at a downstream end (70a, 120a, 220a, 320a, 420a) of the compressible tube, the downstream fluid channel cross-sectional area being greater than the upstream fluid channel cross sectional area;
applying sufficient pressure directed radially inward on the outside walls to compress the compressible tube and create a temporary seal to stop a flow of urine in the fluid channel; and
releasing the pressure to remove the seal and allow urine temporarily blocked by the seal to drain from the fluid area.

21. The method of claim 20, further comprising applying the pressure using a surgical clamping device.

22. The method of claim 21, further comprising employing a hemostat to apply the pressure.

23. The method of claim 20, further comprising selecting the compressible tube (20, 120, 220, 320, 420) to provide a ratio of the downstream fluid channel cross-sectional area to the upstream fluid channel cross-sectional area that accelerates a rate of drainage of urine from the compressible tube when the pressure is released relative to the rate of drainage of urine provided when a compressible tube having a constant fluid channel cross-sectional area is located in the fluid channel between the sample port fitting (16) and the in-line vent (18).

24. The method of claim 23, further comprising selecting the ratio to accelerate the rate of drainage by at least a factor of two.

25. The [[A]] urine collection system (10) of claim 1:
wherein the upstream flow-path diameter of the compressible tube is at least as great as the downstream flow-path diameter of the sampling port fitting.

26. The urine collection system (10) of claim 25, wherein the in-line vent line (18) includes an upstream end (60) having an upstream flow-path diameter and a downstream end (62) having a downstream flow-path diameter,
wherein the downstream end (70a) of the compressible tube is configured to couple to the upstream end of the in-line vent,
wherein the upstream flow-path diameter of the in-line vent is at least as great as the downstream flow-path diameter of the compressible tube, and
wherein a minimum diameter of a flow-path provided by the in-line vent is no less than the upstream flow-path diameter of the in-line vent.

27. The urine collection system of claim 26, further comprising a drain line (14) including an upstream end (14a) having an upstream flow-path diameter and a downstream end (14b) having a downstream flow-path diameter,
wherein the downstream end (62) of the in-line vent (18) is configured to couple to the upstream end of the drain line,
wherein the upstream flow-path diameter of the drain line is at least as great as the downstream flow-path diameter of the in-line vent, and
wherein a minimum diameter of a flow-path provided by the drain line is no less than the upstream flow-path diameter of the drain line.

28. A method of urine collection with a urine collection system (10) that defines a flow path and comprises components including a sample port fitting (16) defining a first region of a fluid channel for urine flow, an in-line vent (18) located downstream of the sample port fitting and defining a second region of the fluid channel, a compressible tube (20) defining a region of the fluid channel between the sample port fitting and the in-line vent, and a drain line defining a region of the fluid channel between the in-line vent and a collection bag (12), the method comprising:
providing a minimum diameter of the fluid channel defined by each of the sample port fitting, the in-line vent, the compressible tube and the drain line, respectively, at an inlet to each of the respective components; and
providing a diameter of the fluid channel defined at the inlet of each of the respective components to be at least as large as a maximum diameter of the fluid channel defined by the respective component located immediately upstream in the flow path; **characterized by**
providing the compressible tube with a fluid channel (72) having a diameter at an outlet end (20a) of the compressible tube that is greater than a diameter at an inlet end (20b) of the compressible tube.

## Patentansprüche

1. Urinsammelsystem (10), umfassend:
einen Sammelbeutel (12), der ein Flüssigkeitsreservoir mit einem Flüssigkeitseinlass und einem Flüssigkeitsauslass definiert;
eine Leitungsöffnung (18), die vorgelagert zum Sammelbeutel positioniert ist und an den Sammelbeutel fluidgekoppelt ist, wobei die Leitungsöffnung ein Auslassende (62) beinhaltet;
ein Probenahmenanschlussstück (16), das vorgelagert zur Leitungsöffnung positioniert ist und so angepasst ist, dass es an einen Urinkatheter (30) fluidgekoppelt wird; und
einen komprimierbaren Schlauch (20, 120, 220, 320, 420), der einen Flüssigkeitskanal (72, 172, 272, 372, 472) definiert, der das Probenahmenanschlussstück und die Leitungsöffnung fluidverbindet, wobei der komprimierbare Schlauch klemmbar ist, um den Flüssigkeitskanal abzudichten, **dadurch gekennzeichnet, dass**
ein Innendurchmesser des durch den komprimierbaren Schlauch definierten Flüssigkeitskanals an einem nachgelagerten Ende (70a, 120a, 220a, 320a, 420a) des Flüssigkeitskanals größer ist als der Innendurchmesser an einem vorgelagerten Ende (70b, 120b, 220b, 320b, 420b) des Flüssigkeitskanals.

2. Urinsammelsystem (10) nach Anspruch 1, wobei das Auslassende (62) der Leitungsöffnung (18) mit einer Ablaufleitung (14) verbunden ist, und die Ablaufleitung mit dem Sammelbeutel (12) verbunden ist.

3. Urinsammelsystem (10) nach Anspruch 1, wobei der komprimierbare Schlauch (20, 120, 220, 320, 420) einen komprimierbaren Teil beinhaltet, der einen konischen Flüssigkeitskanal definiert, der einen Flüssigkeitsfließweg mit einem zunehmenden Durchmesser in einer nachgelagerten Richtung bereitstellt.

4. Urinsammelsystem (10) nach Anspruch 3, wobei eine Innenfläche des komprimierbaren Schlauchs (20, 120, 220, 320, 420), der den komprimierbaren Teil beinhaltet, der einen konischen Flüssigkeitskanal definiert, mit einer nicht herauslösbaren, gleitfähigen Beschichtung behandelt wird.

5. Urinsammelsystem (10) nach Anspruch 3, wobei der komprimierbare Schlauch (20, 120, 220, 320, 420) mit dem komprimierbaren Teil, der einen konischen Flüssigkeitskanal definiert, gefärbt ist, um eine einfach zu identifizierende Klemmfläche bereitzustellen.

6. Urinsammelsystem (10) nach Anspruch 5, wobei der Flüssigkeitskanal (72, 172, 272, 372, 472) des komprimierbaren Schlauchs (20, 120, 220, 320, 420) einen vorgelagerten zylindrischen Teil und einen nachgelagerten zylindrischen Teil beinhaltet, und
wobei der komprimierbare Teil, der einen konischen Flüssigkeitskanal definiert, sich zwischen dem vorgelagerten zylindrischen Teil und dem nachgelagerten zylindrischen Teil befindet.

7. Urinsammelsystem (10) nach Anspruch 1, wobei der Durchmesser des Flüssigkeitskanals (72, 172, 272, 372, 472) des komprimierbaren Schlauchs (20, 120, 220, 320, 420) gleichmäßig vom vorgelagerten Ende zum nachgelagerten Ende zunimmt.

8. Urinsammelsystem (10) nach Anspruch 1, wobei der komprimierbare Schlauch (320) eine Vielzahl von komprimierbaren Teilen (370c, 370e) beinhaltet, die jeweils konische Flüssigkeitskanäle definieren, wobei jeder der konischen Flüssigkeitskanäle einen Flüssigkeitsfließkanal mit einem zunehmenden Durchmesser in einer nachgelagerten Richtung bereitstellt.

9. Urinsammelsystem (10) nach Anspruch 8, wobei der komprimierbare Schlauch (320) einen entsprechenden zylindrischen Teil (370d) beinhaltet, der einen zylindrisch geformtem Flüssigkeitsfließkanal definiert, um jede der Vielzahl von komprimierbaren Teilen zu trennen.

10. Vorrichtung zum Abdichten eines Flüssigkeitskanals in einem Urinsammelsystem (10) mit jeweils einem Probenahmenanschlussstück (16), das für das Ermöglichen von Zugang zum Flüssigkeitskanal konfiguriert ist, und einer Öffnung (18), die zum Koppeln des Flüssigkeitskanals an atmosphärischen Druck konfiguriert ist, wobei die Vorrichtung Folgendes umfasst:
einen komprimierbaren Schlauch (20, 120, 220, 320, 420) mit einem ersten Ende (70a, 120a, 220a, 320a, 420a), konfiguriert zum Koppeln an ein Auslassende (46) des Probenahmenanschlussstücks, wobei das erste Ende einen ersten Innendurchmesser hat, und einem zweiten Ende (70b, 120b, 220b, 320b, 420b), konfiguriert zum Koppeln an ein Einlassende (60) der Öffnung, wobei das zweite Ende einen zweiten Innendurchmesser hat, und wobei der komprimierbare Schlauch den Flüssigkeitskanal zwischen dem Auslassende des Probenahmenanschlussstücks und dem vorgelagerten Ende der Öffnung definiert,
wobei der komprimierbare Schlauch zum Komprimieren konfiguriert ist, um den Fluss der Flüssigkeit in dem Flüssigkeitskanal als Reaktion auf den Klemmdruck, der an einem Teil des komprimierbaren Schlauchs zwischen dem ersten Ende und zweiten Ende angelegt wird, zu stoppen, **dadurch gekennzeichnet, dass**
der zweite Innendurchmesser größer als der erste Innendurchmesser ist.

11. Vorrichtung nach Anspruch 10, wobei das erste Ende (70a, 120a, 220a, 320a, 420a) einen Außendurchmesser beinhaltet, der für die Aufnahme innerhalb des Probenahmenanschlussstücks (16) bemessen ist, um eine luftdichte Verbindung mit dem ersten Ende, das an das Probenahmenanschlussstück gekoppelt ist, bereitzustellen, und
wobei der zweite Innendurchmesser so bemessen ist, dass er um eine Außenfläche des vorgelagerten Endes der Öffnung (18) passt, um eine luftdichte Verbindung mit dem zweiten Ende, das an die Öffnung gekoppelt ist, bereitzustellen.

12. Vorrichtung nach Anspruch 10, wobei der komprimierbare Schlauch (20, 120, 220, 320, 420) zumindest einen konischen Teil (70c, 170c, 270c, 370c, 370e, 470a) beinhaltet.

13. Vorrichtung nach Anspruch 12, wobei der komprimierbare Schlauch (120) einen kontinuierlichen Konus vom ersten Ende zum zweiten Ende beinhaltet.

14. Vorrichtung nach Anspruch 12, wobei der komprimierbare Schlauch (320) eine Vielzahl von konischen Teilen (370c, 370e) beinhaltet.

15. Vorrichtung nach Anspruch 14, wobei der komprimierbare Schlauch (320) einen Bereich mit konstantem Durchmesser (370d) zwischen und unmittelbar neben zwei der Vielzahl von konischen Teilen beinhaltet.

16. Vorrichtung nach Anspruch 12, wobei der komprimierbare Schlauch (20, 120, 220, 320, 420) so bemessen und konfiguriert ist, dass die Öffnung (18) auf luftdichte Weise gekoppelt wird, während zumindest ein maximaler Innendurchmesser des komprimierbaren Schlauchs beibehalten wird.

17. Vorrichtung nach Anspruch 10, wobei der zweite Innendurchmesser um zumindest 1 mm größer als der erste Innendurchmesser ist.

18. Vorrichtung nach Anspruch 10, wobei der erste Innendurchmesser ein Mindestdurchmesser des durch den komprimierbaren Schlauch (20, 120, 220, 320, 420) definierten Flüssigkeitskanals ist.

19. Vorrichtung nach Anspruch 18, wobei der erste Innendurchmesser so konfiguriert ist, dass er zumindest gleich einem Durchmesser des durch das Probenahmenanschlussstück (16) definierten Flüssigkeitskanals ist.

20. Verfahren zum temporären Abdichten eines Flüssigkeitskanals in einem Urinsammelsystem (10) mit einem Probenahmenanschlussstück (16) und einer Leitungsöffnung (18), die dem Probenahmenanschlussstück im Flüssigkeitskanal nachgelagert ist, wobei das Verfahren Folgendes umfasst:
das Positionieren eines komprimierbaren Schlauchs (20, 120, 220, 320, 420) im Flüssigkeitskanal zwischen dem Probenahmenanschlussstück (16) und der Leitungsöffnung (18), wobei der komprimierbaren Schlauch Außenwände, einen vorgelagerten Flüssigkeitskanalquerschnittsbereich am vorgelagerten Ende (70b, 120b, 220b, 320b, 420b) des komprimierbaren Schlauchs und einen nachgelagerten Flüssigkeitskanalquerschnittsbereich am nachgelagerten Ende (70a, 120a, 220a, 320a, 420a) des komprimierbaren Schlauchs beinhaltet, wobei der nachgelagerte Flüssigkeitskanalquerschnittsbereich größer als der vorgelagerte Flüssigkeitskanalquerschnittsbereich ist;
das Anlegen von ausreichend Druck, der radial nach innen an den Außenwänden gerichtet ist, um den komprimierbaren Schlauch zu komprimieren und eine temporäre Abdichtung zu schaffen, um einem Fluss von Urin im Flüssigkeitskanal zu stoppen; und
das Lösen des Drucks, um die Abdichtung zu entfernen und es dem temporär durch die Abdichtung blockierten Urin zu ermöglichen, aus dem Flüssigkeitsbereich abzulaufen.

21. Verfahren nach Anspruch 20, weiterhin umfassend das Anlegen des Druckes unter Verwendung einer chirurgischen Klemmvorrichtung.

22. Verfahren nach Anspruch 21, weiterhin umfassend das Verwenden eines Hämostats, um den Druck anzulegen.

23. Verfahren nach Anspruch 20, weiterhin umfassend das Auswählen des komprimierbaren Schlauchs (20, 120, 220, 320, 420), um ein Verhältnis des nachgelagerten Flüssigkeitskanalquerschnittsbereichs zum vorgelagerten Flüssigkeitskanalquerschnittsbereich bereitzustellen, das eine Ablaufgeschwindigkeit des Urins aus dem komprimierbaren Schlauch beschleunigt, wenn der Druck gelöst wird, relativ zur Ablaufgeschwindigkeit von Urin, die bereitgestellt wird, wenn sich ein komprimierbarer Schlauch mit einem konstanten Flüssigkeitskanalquerschnittsbereich im Flüssigkeitskanal zwischen dem Probenahmenanschlussstück (16) und der Leitungsöffnung (18) befindet.

24. Verfahren nach Anspruch 23, weiterhin umfassend das Auswählen des Verhältnisses, um die Ablaufgeschwindigkeit um zumindest einen Faktor Zwei zu beschleunigen.

25. Urinsammelsystem (10) nach Anspruch 1:
wobei der Durchmesser des vorgelagerten Fließwegs des komprimierbaren Schlauchs zumindest genauso groß wie der Durchmesser des nachgelagerten Fließwegs des Probenahmenanschlussstücks ist.

26. Urinsammelsystem (10) nach Anspruch 25, wobei die Leitungsöffnungsleitung (18) ein vorgelagertes Ende (60) mit einem Durchmesser des vorgelagerten Fließwegs und ein nachgelagertes Ende (62) mit einem Durchmesser des nachgelagerten Fließwegs beinhaltet,
wobei das nachgelagerte Ende (70a) des komprimierbaren Schlauchs zum Koppeln an das vorgelagerte Ende der Leitungsöffnung konfiguriert ist,
wobei der Durchmesser des vorgelagerten Fließwegs der Leitungsöffnung zumindest genauso groß wie der Durchmesser des nachgelagerten Fließwegs des komprimierbaren Schlauchs ist, und
wobei ein Mindestdurchmesser eines Fließwegs, der durch die Leitungsöffnung bereitgestellt wird, nicht kleiner als der Durchmesser des vorgelagerten Fließwegs der Leitungsöffnung ist.

27. Urinsammelsystem nach Anspruch 26, weiterhin umfassend eine Ablaufleitung (14), die ein vorgelagertes Ende (14a) mit einem Durchmesser des vorgelagerten Fließwegs und einem nachgelagerten Ende (14b) mit einem Durchmesser des nachgelagerten Fließwegs beinhaltet,
wobei das nachgelagerte Ende (62) der Leitungsöffnung (18) zum Koppeln an das vorgelagerte Ende der Ablaufleitung konfiguriert ist,
wobei der Durchmesser des vorgelagerten Fließwegs der Ablaufleitung zumindest genauso groß wie der Durchmesser des nachgelagerten Fließwegs der Leitungsöffnung ist, und
wobei ein Mindestdurchmesser eines Fließwegs, der durch die Ablaufleitung bereitgestellt wird, nicht kleiner als der Durchmesser des vorgelagerten Fließwegs der Ablaufleitung ist.

28. Verfahren der Urinsammlung mit einem Urinsammelsystem (10), das einen Fließweg definiert und Komponenten umfasst, einschließlich eines Probenahmenanschlussstücks (16), das einen ersten Bereich eines Flüssigkeitskanals für Urinfluss definiert, einer Leitungsöffnung (18), die sich nachgelagert zum Probenahmenanschlussstück befindet und einen zweiten Bereich des Flüssigkeitskanals definiert, eines komprimierbaren Schlauchs (20), der einen Bereich des Flüssigkeitskanals zwischen dem Probenahmenanschlussstück und der Leitungsöffnung definiert, und einer Ablaufleitung, die einen Bereich des Flüssigkeitskanals zwischen der Leitungsöffnung und einem Sammelbehälter (12) definiert, wobei das Verfahren Folgendes umfasst:
das Bereitstellen eines Mindestdurchmessers des Flüssigkeitskanals, definiert durch jeden aus dem Probenahmenanschlussstück, der Leitungsöffnung, dem komprimierbaren Schlauch und der Ablaufleitung, an einem Einlass zu jeder der entsprechenden Komponenten; und
das Bereitstellen eines Durchmessers des Flüssigkeitskanals, der am Einlass von jeder der entsprechenden Komponenten so definiert ist, dass er zumindest so groß wie der Maximaldurchmesser des Flüssigkeitskanals ist, der durch die entsprechende Komponente unmittelbar vorgelagert im Fließweg definiert wird; **gekennzeichnet durch**
das Bereitstellen des komprimierbaren Schlauchs mit einem Flüssigkeitskanal (72) mit einem Durchmesser an einem Auslassende (20a) des komprimierbaren Schlauchs, der größer als ein Durchmesser an einem Einlassende (20b) des komprimierbaren Schlauchs ist.

## Revendications

1. Système de collecte d'urine (10) comprenant :
une poche de recueil (12) définissant un réservoir à liquide comprenant un orifice d'entrée du liquide et un orifice de sortie du liquide ;
un évent en ligne (18) situé en amont de la poche de recueil et couplé de manière fluidique à la poche de recueil, l'évent en ligne comprenant une extrémité de sortie (62) ;
un raccord pour point de prélèvement (16) situé en amont de l'évent en ligne et conçu pour être couplé de manière fluidique à un cathéter urinaire (30) ; et
un tube compressible (20, 120, 220, 320, 420) définissant un canal de liquide (72, 172. 272, 372, 472) reliant de manière fluidique le raccord pour point de prélèvement et l'évent en ligne, le tube compressible pouvant être comprimé de manière à obstruer le canal de liquide, **caractérisé en ce que**
un diamètre interne du canal de liquide défini par le tube compressible est supérieur à une extrémité en aval (70a, 120a, 220a, 320a, 420a) du canal de liquide au diamètre interne à une extrémité en amont (70b, 120b, 220b, 320b, 420b) du canal de liquide.

2. Système de collecte d'urine (10) selon la revendication 1, dans lequel l'extrémité de sortie (62) de l'évent en ligne (18) est reliée à un conduit de drainage (14) et le conduit de drainage est relié à la poche de recueil (12).

3. Système de collecte d'urine (10) selon la revendication 1, dans lequel le tube compressible (20, 120, 220, 320, 420) comprend une portion compressible qui définit un canal de liquide diminué produisant une trajectoire d'écoulement de liquide d'un diamètre croissant dans le sens en aval.

4. Système de collecte d'urine (10) selon la revendication 3, dans lequel une surface interne du tube compressible (20, 120, 220, 320, 420) comprenant la portion compressible qui définit le canal de liquide diminué est traitée avec un revêtement lubrifié non soluble.

5. Système de collecte d'urine (10) selon la revendication 3, dans lequel le tube compressible (20, 120, 220, 320, 420) comprenant la portion compressible qui définit le canal de liquide diminué est un tube de couleur facilitant le repérage de la surface à comprimer.

6. Système de collecte d'urine (10) selon la revendication 5, dans lequel le canal de liquide (72, 172, 272, 372, 472) du tube compressible (20, 120, 220, 320, 420) comprend une portion cylindrique en amont et une portion cylindrique en aval, et
dans lequel la portion compressible qui définit le canal de liquide diminué est située entre la portion cylindrique en amont et la portion cylindrique en aval.

7. Système de collecte d'urine (10) selon la revendication 1, dans lequel le diamètre du canal de liquide (72, 172, 272, 372, 472) du tube compressible (20, 120, 220, 320, 420) augmente uniformément de l'extrémité en amont à l'extrémité en aval.

8. Système de collecte d'urine (10) selon la revendication 1, dans lequel le tube compressible (320) comprend une pluralité de portions compressibles (370c, 370e) qui définissent chacune un canal de liquide diminué, chacun des canaux de liquide diminués produisant un canal d'écoulement de liquide d'un diamètre croissant dans le sens en aval.

9. Système de collecte d'urine (10) selon la revendication 8, dans lequel le tube compressible (320) comprend une portion cylindrique (370d) qui définit un canal d'écoulement de liquide de forme cylindrique pour séparer chacune une pluralité de portions compressibles.

10. Appareil de blocage d'un canal de liquide dans un système de collecte d'urine (10) comprenant un raccord pour point de prélèvement (16) conçu pour permettre l'accès au canal de liquide et un évent (18) conçu pour coupler le canal de fluide à la pression atmosphérique, l'appareil comprenant :
un tube compressible (20, 120, 220, 320, 420) ayant une première extrémité (70a, 120a, 220a, 320a, 420a) conçue pour être couplée à une extrémité de sortie (46) du raccord pour point de prélèvement, la première extrémité ayant un premier diamètre intérieur, et une deuxième extrémité (70b, 120b, 220b, 320b, 420b) conçue pour être couplée à une extrémité d'entrée (60) de l'évent, la deuxième extrémité ayant un deuxième diamètre intérieur, le tube compressible définissant le canal de fluide entre l'extrémité de sortie du raccord pour point de prélèvement et l'extrémité en amont de l'évent,
dans lequel le tube compressible est conçu pour comprimer et stopper l'écoulement du liquide dans le canal de liquide suite à la pression exercée sur une portion du tube compressible située entre la première extrémité et la deuxième extrémité, **caractérisé en ce que**
le deuxième diamètre intérieur est supérieur au premier diamètre intérieur.

11. Appareil selon la revendication 10, dans lequel la première extrémité (70a, 120a, 220, 320a, 420a) comprend un diamètre externe dimensionné pour être inséré dans le raccord pour point de prélèvement (16) et produire un joint étanche à l'air avec la première extrémité couplée au raccord pour point de prélèvement et
dans lequel le deuxième diamètre intérieur est dimensionné pour être ajusté autour d'une surface extérieure de l'extrémité en amont de l'évent (18) pour produire un joint étanche à l'air avec la deuxième extrémité couplée à l'évent.

12. Appareil selon la revendication 10, dans lequel le tube compressible (20, 120, 220, 320, 420) comprend au moins une portion diminuée (70c, 170c, 270c, 370c, 370e, 470a).

13. Appareil selon la revendication 12, dans lequel le tube compressible (120) comprend une diminution continue de la première extrémité à la deuxième extrémité.

14. Appareil selon la revendication 12, dans lequel le tube compressible (320) comprend une pluralité de portions diminuées (370c, 370e).

15. Appareil selon la revendication 14, dans lequel le tube compressible (320) comprend une zone de diamètre constant (370d) située entre et immédiatement adjacente à deux de la pluralité des portions diminuées.

16. Appareil selon la revendication 12, dans lequel le tube compressible (20, 120, 220, 320, 420) est dimensionné et conçu pour être couplé à l'évent (18) de manière étanche à l'air tout en maintenant au moins un diamètre intérieur maximal du tube compressible.

17. Appareil selon la revendication 10, dans lequel le deuxième diamètre intérieur dépasse le premier diamètre intérieur d'au moins 1 mm.

18. Appareil selon la revendication 10, dans lequel le premier diamètre intérieur est un diamètre minimal du canal de liquide défini par le tube compressible (20, 120, 330, 320, 420).

19. Appareil selon la revendication 18, dans lequel le premier diamètre intérieur est conçu pour être au moins égal au diamètre du canal de liquide défini par le raccord pour point de prélèvement (16).

20. Procédé permettant d'obstruer provisoirement un canal de liquide dans un système de collecte d'urine (10) comprenant un raccord pour point de prélèvement (16) et un évent en ligne (18) situé en aval du raccord pour point de prélèvement du canal de liquide, le procédé comprenant les étapes consistant à :
installer un tube compressible (20, 120, 220, 320, 420) dans le canal de liquide entre le raccord pour point de prélèvement (16) et l'évent en ligne (18), le tube compressible comprenant des parois extérieures, une section de canal de liquide en amont située à une extrémité en amont (70b, 120b, 220b, 320b, 420b) du tube compressible et une section de canal de liquide en aval située à une extrémité en aval (70a, 120a, 220a, 320a, 420a) du tube compressible, la section du canal de liquide en aval étant supérieure à la section du canal de liquide en amont ;
exercer une force suffisante directement, radialement et vers l'intérieur sur les parois extérieures pour comprimer le tube compressible et créer un blocage provisoire pour stopper l'écoulement de l'urine dans le canal de liquide ; et
relâcher la pression pour supprimer le blocage et permettre à l'urine provisoirement bloquée par la force de compression d'être évacuée de la zone de liquide.

21. Procédé selon la revendication 20, comprenant en outre l'étape consistant à exercer la pression avec un dispositif de clampage chirurgical.

22. Procédé selon la revendication 21, comprenant en outre l'étape consistant à utiliser une pince hémostatique pour exercer la pression.

23. Procédé selon la revendication 20, comprenant en outre l'étape consistant à sélectionner le tube compressible (20, 120, 220, 320, 420) de manière à obtenir un rapport de la section du canal de liquide en aval sur la section du canal de liquide en amont qui accélère la vitesse de drainage de l'urine depuis le tube compressible lorsque la pression est relâchée par rapport à la vitesse de drainage de l'urine produite quand un tube compressible à section de canal de liquide constante est situé dans le canal de liquide entre le raccord pour point de prélèvement (16) et l'évent en ligne (18).

24. Procédé selon la revendication 23, comprenant en outre l'étape consistant à sélectionner le rapport entre les deux sections de manière à accélérer la vitesse de drainage d'un facteur d'au moins deux.

25. Système de collecte d'urine [[A]](10) selon la revendication 1 :
dans lequel le diamètre d'écoulement en amont du tube compressible est au moins aussi grand que le diamètre d'écoulement en aval du raccord pour point de prélèvement.

26. Système de collecte d'urine (10) selon la revendication 25, dans lequel l'évent en ligne (18) comprend une extrémité en amont (60) ayant un diamètre d'écoulement en amont et une extrémité en aval (62) ayant un diamètre d'écoulement en aval,
dans lequel l'extrémité en aval (70a) du tube compressible est conçue pour être couplée à l'extrémité en amont de l'évent en ligne,
dans lequel le diamètre d'écoulement en amont de l'évent en ligne est au moins aussi grand que le diamètre d'écoulement en aval du tube compressible, et
dans lequel un diamètre minimal d'écoulement produit par l'évent en ligne n'est pas inférieur au diamètre d'écoulement en amont de l'évent en ligne.

27. Système de collecte d'urine selon la revendication 26, comprenant en outre un conduit de drainage (14) comprenant une extrémité en amont (14a) ayant un diamètre d'écoulement en amont et une extrémité en aval (14b) ayant un diamètre d'écoulement en aval,
dans lequel l'extrémité en aval (62) de l'évent en ligne (18) est conçue pour être couplée à l'extrémité en amont du conduit de drainage,
dans lequel le diamètre d'écoulement en amont du conduit de drainage est au moins aussi grand que le diamètre d'écoulement en aval de l'évent en ligne, et
dans lequel un diamètre minimal d'écoulement produit dans le conduit de drainage n'est pas inférieur au diamètre d'écoulement en amont du conduit de drainage.

28. Procédé de collecte d'urine avec un système de collecte d'urine (10) qui définit une trajectoire d'écoulement et comprend des composants comprenant un raccord pour point de prélèvement (16) définissant une première zone d'un canal de liquide pour l'écoulement de l'urine, un évent en ligne (18) situé en aval du raccord pour point de prélèvement et définissant une deuxième zone du canal de liquide, un tube compressible (20) définissant une zone du canal de liquide entre le raccord pour point de prélèvement et l'évent en ligne, et un conduit de drainage définissant une zone du canal de liquide entre l'évent en ligne et une poche de recueil (12), le procédé comprenant :
la fourniture d'un diamètre minimal du canal de liquide défini par le raccord pour point de prélèvement, l'évent en ligne, le tube compressible et le conduit de drainage, respectivement, au port d'entrée de chacun des composants ; et
la fourniture d'un diamètre du canal de liquide défini au port d'entrée de chacun des composants respectifs pour être aussi large qu'un diamètre maximal du canal de liquide défini par le composant respectif situé immédiatement en amont dans la trajectoire d'écoulement ; **caractérisé par**
la fourniture du tube compressible avec un canal de liquide (72) ayant un diamètre à l'extrémité de sortie (20a) du tube compressible qui est supérieur au diamètre à l'extrémité d'entrée (20b) du tube compressible.
